Europäisches Patentamt

European Patent Office

Office européen des brevets

(19)

(11) Publication number: **0 231 819 B1**

# EUROPEAN PATENT SPECIFICATION

(12)

(45) Date of publication of patent specification: **01.04.92**   (51) Int. Cl.⁵: **A61K 37/02**

(21) Application number: **87100809.0**

(22) Date of filing: **21.01.87**

The file contains technical information submitted after the application was filed and not included in this specification

(54) **Pharmaceutical agent for the treatment of myelogenous leukemia.**

(30) Priority: **22.01.86 JP 10281/86**

(43) Date of publication of application:
**12.08.87 Bulletin 87/33**

(45) Publication of the grant of the patent:
**01.04.92 Bulletin 92/14**

(84) Designated Contracting States:
**AT BE CH DE ES FR GB IT LI NL SE**

(56) References cited:
**EP-A- 0 169 566**
**EP-A- 0 215 126**

**CELL, vol. 43, no. 1, 1985, pages 5-6; D. METCALF: "The granulocyte-macrophage colony stimulating factors"**

**SYMPOSIUM ON FUNDAMENTAL CANCER RESEARCH, 1985, pages 147-158; M.A.S. MOORE et al.: "Myeloid and erythroid stem cells: regulation in normal and neoplastic states"**

(73) Proprietor: **Chugai Seiyaku Kabushiki Kaisha 5-1, 5-chome, Ukima Kita-ku Tokyo(JP)**

(72) Inventor: **Tamura, Masahiko Sahoda Roiyaruhaitsu 302 Kuji 14 Takatsu-ku Kawasaki-shi Kanagawa-ken(JP)**
Inventor: **Hattori, Kunihiro Chugai inokashiraryo, 2-20-9, Inokashira Mitaka-shi Tokyo(JP)**

(74) Representative: **Vossius & Partner Siebertstrasse 4 P.O. Box 86 07 67 W-8000 München 86(DE)**

NATURE, vol. 314, 18th April 1985, pages 625-628; N.A. NICOLA et al.: "Identification of the human analogue of a regulator that induces differentiation in murine leukaemic cells"

PROC. NATL. ACAD. SCI., vol. 79, July 1982, pages 4347-4351; J. LOTEM et al.: "Mechanisms that uncouple growth and differentiation in myeloid leukemia cells: restoration of requirement for normal growth-inducing protein without restoring induction of differentiation-inducing protein"

THE JOURNAL OF BIOLOGICAL CHEMISTRY, vol. 258, no. 14, 25th July 1983, pages 9017-9023, US; N.A. NICOLA et al.: "Purification of a factor inducing differentiation in murine myelomonocyte leukemia cells"

LEUKEMIA RESEARCH, vol. 7, no. 2, 1983, pages 117-132, Pergamon Press Ltd, GB; D. METCALF: "Clonal analysis of the response of HL60 human myeloid leukemia cells to biological regulators"

CANCER RESEARCH, vol. 44, no. 11, November 1984, pages 5169-5175; S.L. PERKINS et al.: "Human myeloid leukemic cell (HL-60) autostimulator: relationship to colony-stimulating factor"

SCIENCE, vol. 232, no. 4746, 4th April 1986, pages 61-65; L.M. SOUZA et al.: "Recombinant human granulocyte colony-stimulating factor: effects on normal and leukemic myeloid cells"

**Description**

The present invention relates to a pharmaceutical agent for the treatment of myelogenous leukemia that contains a human granulocyte colony stimulating factor (hereinafter abbreviated as G-CSF) as the active ingredient.

In spite of the development of drugs as typified by chemotherapeutics or the advances in therapeutic regimens such as bone marrow transplantation, leukemia still remains a disease that is difficult to completely cure as is manifested by its mortality rate that has not decreased for the past 20 years [see Igaku no Ayumi, 128, 13, 1984 (hereinafter abbreviated as Ayumi), 867 - 873]. On the other hand, efforts to develop anti-leukemic drugs are being made at a remarkable pace and the rate of complete remission of adult acute myelogenous leukemia (AML) in patients who have received a treatment by multiple chemotherapeutics (complete remission of this disease is indicated by normalization in the quality and quantity of blood cells in peripheral blood and bone marrow and by the disappearance of the subjective symptoms and objective physical findings due to leukemia) has reportedly reached levels of 80% or more (see Ayumi, 994 - 998). Unfortunately, however, the efforts so far made to achieve complete healing of AML have proved very unrewarding as evidenced by its high mortality rate and there is no efficacious drug available for the treatment of chronic myelogenous leukemia (CML) (see Ayumi, 1005 - 1011).

Under these circumstances, studies are being undertaken to develop a new type of leukemia-treating drug that utilises biological response moderators (BRM) such as immunotherapeutics (Ayumi, 1050 - 1055), differentiation inducers (Ayumi, 1059 - 1063) and interferons (Ayumi, 1056 - 1058). However, no drug has been proposed to date that is capable of complete healing of leukemia.

An interesting finding has recently been reported; a substance capable of inducing the differentiation of mouse myelogenous leukemia cell, WEHI-3B, was purified from the supernatant of the culture of the lung tissue of mice and it was found to be identical with a mouse G-CSF (Nicola, N.A. et al.; J. Biol. Chem., 258, 9017 - 9023, 1983). This G-CSF is a BRM that acts on granulocyte precursor cells to promote their differentiation and growth to granulocytes (see, for example, Metcalf et al.; Exp. Hematol., 1, 185, 1973).

The present inventors have conducted intensive studies on human G-CSF and filed patent applications on their success in achieving large-scale preparation of a pure human G-CSF (see EP No. 85 10 9336.9 (EP-A 169566), EP No. 86 11 3671.1 (EP-A 217404) and EP No. 86 90 1138.7 (EP-A 215126, WO 8604605). One of the CSFs prepared by the present inventors is a human G-CSF derived from CHU-2. When this CSF was administered to mice, the number of mature neutrophiles in peripheral blood showed a marked increase (see Experiment 1 described later in this specification). An experiment was also conducted to investigate the efficacy of G-CSF for treating radiation-induced leukemia in SJL/J mouse models. A significant life-span prolonging effect was achieved by G-CSF (see Experiment 2). The SJL/J mice are models in which leukemia was induced by irradiation and they enable more reliable simulation of leukemia than conventional models in which leukemia cell lines are transplanted intraperitoneally. The SJL/J mice are highly prone to develop myelogenous leukemia (15 - 20% incidence upon exposure to irradiation of total dose of 300 R). In Experiment 2, it was also demonstrated that administration of G-CSF caused an increase in the proportion of mature neutrophiles and this was confirmed by classification of peripheral neutrophilic cells on the basis of their maturity. These mature neutrophiles may have originated from (1) differentiated leukemic cells or from (2) the differentiation and growth of normal cells that remained in leukemic mice. Whichever the case may be, the life-span prolonging effect of G-CSF is most probably due to its ability to increase the number of peripheral mature neutrophiles in leukemic mice. Since the results of Experiment 2 were obtained from models that closely simulate the development of leukemia, it may be safely concluded that they demonstrate the usefulness of G-CSF as a leukemia treating agent.

The present invention has been accomplished on the basis of the aforementioned findings.

The present invention provides the use of a human G-CSF for the preparation of a pharmaceutical agent for the treatment of myelogenous leukemia.

The human G-CSF used as the active ingredient of the pharmaceutical agent of the present invention may be derived from any origin that is capable of producing an isolated human G-CSF of high purity. It is preferable to use the following two types of human G-CSF that were obtained by the methods on which patent applications were previously applied by the present inventors:

(I) human G-CSF having the following physicochemical properties:

i) molecular weight: 19,000 ± 1,000 as measured by electrophoresis through a sodium dodecylsulphate - polyacrylamide gel;

ii) isoelectric point: having at least one of the three isoelectric points, pI = 5.5 ± 0.1, pI = 5.8 ± 0.1, and pI = 6.1 ± 0.1;

iii) ultraviolet absorption: having a maximum absorption at 280 nm and a minimum absorption at 250 nm;

iv) amino acid sequence of the 2l residues from N terminus:

```
H₂N - Thr - Pro - Leu - Gly - Pro - Ala - Ser - Ser -

Leu - Pro - Gln - Ser - Phe - Leu - Leu - Lys - Cys -

Leu - Glu - Gln - Val -
```

(2) a human G-CSF having a polypeptide represented by all or part of the following amino acid sequence:

```
(Met)ₙThr  Pro  Leu  Gly  Pro  Ala  Ser  Ser  Leu  Pro
Gln  Ser  Phe  Leu  Leu  Lys  Cys  Leu  Glu  Gln  Val
Arg  Lys  Ile  Gln  Gly  Asp  Gly  Ala  Ala  Leu  Gln
```

```
Glu  Lys  X    Cys  Ala  Thr  Tyr  Lys  Leu  Cys  His
Pro  Glu  Glu  Leu  Val  Leu  Leu  Gly  His  Ser  Leu
Gly  Ile  Pro  Trp  Ala  Pro  Leu  Ser  Ser  Cys  Pro
Ser  Gln  Ala  Leu  Gln  Leu  Ala  Gly  Cys  Leu  Ser
Gln  Leu  His  Ser  Gly  Leu  Phe  Leu  Tyr  Gln  Gly
Leu  Leu  Gln  Ala  Leu  Glu  Gly  Ile  Ser  Pro  Glu
Leu  Gly  Pro  Thr  Leu  Asp  Thr  Leu  Gln  Leu  Asp
Val  Ala  Asp  Phe  Ala  Thr  Thr  Ile  Trp  Gln  Gln
Met  Glu  Glu  Leu  Gly  Met  Ala  Pro  Ala  Leu  Gln
Pro  Thr  Gln  Gly  Ala  Met  Pro  Ala  Phe  Ala  Ser
Ala  Phe  Gln  Arg  Arg  Ala  Gly  Gly  Val  Leu  Val
Ala  Ser  His  Leu  Gln  Ser  Phe  Leu  Glu  Val  Ser
Tyr  Arg  Val  Leu  Arg  His  Leu  Ala  Gln  Pro
```

(where X is Leu or Leu-Val-Ser-Glu; and n is 0 or 1).

Most preferably, either of the two types of human G-CSF takes on the form of glycoprotein having a sugar chain portion.

The G-CSF of type (1) may be prepared by either of the methods described in EP No. 85 10 9336.9 (EP-A 169566) and EP No. 86 11 3671.1 (EP-A 217404). The former application describes a method of isolating the desired human G-CSF from the supernatant of the culture of a cell strain, CHU-1, that was derived from human oral cavity cancer and which has been deposited with Collection Nationale de Cultures de Microorganismes, Institut Pasteur, France under C.N.C.M. Accession Number I-315. The latter application describes a method of isolating the desired human G-CSF from the supernatant of the culture of a cell strain, CHU-2, that was also derived from human oral cavity cancer and which has been deposited with C.N.C.M. under Accession Number I-483. For further details of the two methods, see the specifications of the respective applications.

The G-CSF of type (2) may be prepared by either of the methods described in EP No. 86 90 1138.7 (EP-A 215126). All of these methods rely on "DNA recombinant technology". The methods described in the first two applications use E. coli and other procaryotic cells as host cells, and those given in the other two applications employ animal cells as host cells. For further details of these methods, see the specifications of the respective applications.

The most desirable type of G-CSF which assumes the form of a glycoprotein having a sugar chain portion can be produced by the method using animal cells as hosts.

The human G-CSF obtained by either of the methods outlined above may be stored in a frozen state or after being dehydrated by such means as freeze-drying or vacuum drying. If desired, the human G-CSF may be dissolved in an appropriate buffer, followed by aseptic filtration through a Millipore filter or any other suitable means to formulate an injection.

The pharmaceutical agent for the treatment of myelogenous leukemia of the present invention may contain the pharmaceutical carrier or excipient necessary to assist in its formulation in a dosage form suitable for administration to humans. If desired, a stabilizer and an anti-adsorption agent may also be incorporated in this agent.

The level of dosage and the frequency of administration of the human G-CSF in the pharmaceutical agent of the present invention may be determined in consideration of the severity of the disease to be treated; typically, a dosage containing 0.l - 500 $\mu$g, preferably 5 - l00 $\mu$g, of human G-CSF may be administered to an adult at a frequency of one to seven times a week.

The pharmaceutical agent of the present invention which is intended to be used as a curative for myelogenous leukemia is effective not only for increasing the number of peripheral mature neutrophiles in patients with myelogenous leukemia but also for prolonging their lives. Therefore, the present invention will hold increased promise for the treatment of myelogenous leukemia which has been impossible to cure completely by conventional therapeutic drugs or regimens.

Examples

The following referentce example, experimental examples and working examples are provided for the purpose of illustrating the preparation of G-CSF, its pharmacological effects and its formulation in various dosage forms, respectively, but it should be understood that the scope of the present invention is by no means limited by these examples.

Referentce Example: The Preparation of human G-CSF using animal cells (mouse C127 cells)

Plasmid PTN-V2 was obtained by the procedures described in Examples 1 - 12 of Japanese Patent Application No. 269456/1985 (EP-A 215126), and subsequently treated with BamHI as follows. Twenty micrograms of the plasmid pTN-V2 was dissolved in 100 $\mu$l of a reaction solution [10 mM Tris-HCl (pH 8.0), 7 mM $MgCl_2$, 100 mM NaCl, 2 mM 2-mercaptoethanol and 0.01% BSA] and treated with 20 units of BamHI (Takara Shuzo Co., Ltd.), followed by treatments with phenol and ether, and precipitation with ethanol.

Mouse C127 cells were grown in a Dulbecco's minimal essential medium containing 10% bovine foetal serum (Gibco). The C127 cells growing on plates (5 cm$^{\emptyset}$) were transformed with 10 $\mu$g per plate of the separately prepared DNA by the calcium phosphate procedure [see Haynes, J. & Weissmann, C., Nucleic Acids Res., 11, 687 - 706 (1983)]. After treatment with glycerol, the cells were incubated at 37°C for 12 hours.

The incubated cells were transferred onto three fresh plates (5 cm$^{\emptyset}$) and the media changed twice a week. At day 16, the foci were transferred onto fresh plates and subjected to serial cultivation on a Dulbecco's minimal essential medium containing 10% bovine foetal serum (Gibco), so as to select clones having high G-CSF production rate. These clones produced G-CSF at a level of approximately 1 mg/l.

For the methods of recovering, purifying and assaying the so obtained G-CSF, see the pertinent Examples shown in the specification of Japanese Patent Application No. 269456/1985(EP-A 215126).

Experiment I: Increase in the number of peripheral mature neutrophiles upon G-CSF administration

C57BL mice (male, 8-week-old) were divided into two groups. 0.1 ml of a control sample (a physiological saline solution containing l% n-propanol and l0% serum from C57BL mice) was administered subcutaneously once a day to one group (control group). To the other group (CSF-treated group), 0.l ml of a CSF sample (a physiological saline solution containing 2.5 $\mu$g of CHU-2 derived G-CSF, l% n-propanol and l0% serum from C57BL mice) was administered subcutaneously once a day. On predetermined days, 4 mice were sampled randomly from each group and the number of leukocytes in blood samples taken from the orbital vein was determined with a micro cell counter (Model CCl80 of Toa Medical Electronics Co., Ltd.) In a separate step, blood smears were prepared and Giemsa staining was conducted in order to determine the proportion of peripheral neutrophiles in 200 leukocytes under the microscope. The number of peripheral neutrophiles in each group was calculated by the following formula:

5

Peripheral neutrophiles = (peripheral leukocyte count) × (proportion of neutrophiles in leukocytes)

In this experiment, no mouse was bled on more than one occasion. A group of C57BL mice to which no injection was given at all were treated by the same procedures to obtain the values for day 0. The results are shown in Table I.

Table 1

| (number of measurements, n = 4) | | |
|---|---|---|
| Days | Peripheral neutrophile count (cells/mm$^3$) | |
| | Control group | CSF treated group |
| 0 | 1213 ± 354 | 1213 ± 354 |
| 2 | 769 ± 219 | 3534 ± 596 |
| 5 | 989 ± 158 | 3867 ± 482** |
| 8 | 889 ± 44 | 2724 ± 557** |
| 11 | 696 ± 81 | 3600 ± 407*** |
| 15 | 639 ± 95 | 5020 ± 326*** |
| P: ***<0.001<**<0.01<*<0.05 | | |

As Table 1 shows, the human G-CSF derived from CHU-2 has the ability to increase the number of mature neutrophiles in peripheral blood.

Experiment 2: Anti-leukemic effect of G-CSF in SJL/J leukemic mice.

SJL/J mice (male, 7-week-old) were exposed to irradiation of total dose of 300 R. For a subsequent period of 120 days, blood samples were taken as in Experiment 1 once every 30 days, and once every 10 days in the following period. The numbers of peripheral erythrocytes and leukocytes in each blood sample were counted, and at the same time blood smears were also prepared. Leukemia was considered to have developed if at least two of the following three criteria were satisfied: 1) the peripheral leukocyte count was 30,000 cells/mm$^3$ or more; 2) the peripheral erythrocyte count was 5 x 10$^6$ cells/mm$^3$ or less; and 3) the appearance of leukoblasts in peripheral blood was apparent.

To each of the mice that had developed leukemia, 0.1 ml of the control or G-CSF sample shown in Experiment 1 was administered daily through a subcutaneous route until the mice died. The period for which the mice remained alive following injection of either sample was compared. Another group of leukemic mice were treated in the same manner and blood samples were taken from the orbital vein on predetermined days so as to determine the peripheral leukocyte count and the maturity of neutrophilic granulocytes. All leukemic mice subjected to these tests were found to have experienced swelling of spleen and infiltration of leukemic cells into spleen, which served as another evidence that these mice were truly leukemic. The test results are shown below in (I) and (2) (Table 2).

(I) Days of survival

Control group: 9 ± I.47

CSF treated group: 28.75 ± I.93

(number of measurements, n = 4; P < 0.00I)

Table 2

(2)

| | CSF treated mice | | | | | | | | Control mice | | | |
|---|---|---|---|---|---|---|---|---|---|---|---|---|
| Days after development of leukemia | -15 | 0 | 4 | 9 | 12 | 18 | 27 | 32 | -14 | 0 | 4 | 8 |
| Polymorphonuclear leukocytes* | 84 | 10 | 9 | 19 | 45 | 43 | 18 | 12 | 88 | 12 | 11 | 10 |
| Band cell metamyelocytes* | 16 | 64 | 50 | 29 | 22 | 38 | 42 | 51 | 12 | 53 | 47 | 52 |
| Myelocytes* | 0 | 18 | 21 | 31 | 23 | 11 | 23 | 20 | 0 | 23 | 25 | 13 |
| Promyelocytes* | 0 | 8 | 20 | 21 | 10 | 8 | 13 | 11 | 0 | 12 | 17 | 8 |
| Myeloblasts* | 0 | 0 | 0 | 0 | 0 | 0 | 4 | 6 | 0 | 0 | 0 | 17 |

* Numbers were determined for 100 neutrophilic cells.

As the data in (I) show, administration of G-CSF was effective in prolonging the life-span of leukemic mice by a significant degree. As is clear from the data in (2) (Table 2) which show the numbers of cells at different stages of maturity of neutrophilic cells, the CSF-treated mice had more polymorphonuclear leukocytes (mature neutrophiles) than the control mice. It is believed that this ability of CSF to increase the number of peripheral mature neutrophiles is responsible for the life-span prolonging effect of G-CSF. It is worth particular attention that the results of Experiment 2 were obtained with an animal model that would closely simulate the development of leukemia in the actual case.

Example l

The human G-CSF prepared in the Reference Example was rendered germ-free and frozen at -20°C. The frozen fraction was worked up to prepare an injection.

Example 2

The human G-CSF prepared in the Reference Example was aseptically charged in 5 ml portions in l0 ml vials and freeze-dried at -20°C, with the vials being subsequently closed with rubber stopper. The so obtained freeze-dried products were worked up to prepare an injection.

**Claims**

1. The use of human granulocyte colony stimulating factor for preparing a pharmaceutical composition for treating myelogenous leukemia, said human granulocyte colony stimulating factor having the following physicochemical properties:

i) molecular weight: 19,000 ± 1,000 as measured by electrophoresis through a sodium dodecylsulphate - polyacrylamide gel;

ii) isoelectric point: having at least one of the three isoelectric points, $pI = 5.5 \pm 0.1$, $pI = 5.8 \pm 0.1$, and $pI = 6.1 \pm 0.1$;

iii) ultraviolet absorption: having a maximum absorption at 280 nm and a minimum absorption at 250 nm;

iv) amino acid sequence of the 21 residues from N terminus:

$$H_2N - Thr - Pro - Leu - Gly - Pro - Ala - Ser - Ser -$$
$$Leu - Pro - Gln - Ser - Phe - Leu - Leu - Lys - Cys -$$
$$Leu - Glu - Gln - Val -$$

2. The use of human granulocyte colony stimulating factor for preparing a pharmaceutical composition for treating myelogenous leukemia, said human granulocyte colony stimulating factor representing all or part of the amino acid sequence shown below:

8

```
(Met)ₙThr   Pro   Leu   Gly   Pro   Ala   Ser   Ser   Leu   Pro
     Gln   Ser   Phe   Leu   Leu   Lys   Cys   Leu   Glu   Gln   Val
     Arg   Lys   Ile   Gln   Gly   Asp   Gly   Ala   Ala   Leu   Gln
     Glu   Lys    X    Cys   Ala   Thr   Tyr   Lys   Leu   Cys   His
     Pro   Glu   Glu   Leu   Val   Leu   Leu   Gly   His   Ser   Leu
     Gly   Ile   Pro   Trp   Ala   Pro   Leu   Ser   Ser   Cys   Pro
     Ser   Gln   Ala   Leu   Gln   Leu   Ala   Gly   Cys   Leu   Ser
     Gln   Leu   His   Ser   Gly   Leu   Phe   Leu   Tyr   Gln   Gly
     Leu   Leu   Gln   Ala   Leu   Glu   Gly   Ile   Ser   Pro   Glu
     Leu   Gly   Pro   Thr   Leu   Asp   Thr   Leu   Gln   Leu   Asp
     Val   Ala   Asp   Phe   Ala   Thr   Thr   Ile   Trp   Gln   Gln
     Met   Glu   Glu   Leu   Gly   Met   Ala   Pro   Ala   Leu   Gln
     Pro   Thr   Gln   Gly   Ala   Met   Pro   Ala   Phe   Ala   Ser
     Ala   Phe   Gln   Arg   Arg   Ala   Gly   Gly   Val   Leu   Val

     Ala   Ser   His   Leu   Gln   Ser   Phe   Leu   Glu   Val   Ser
     Tyr   Arg   Val   Leu   Arg   His   Leu   Ala   Gln   Pro
```

(where X is Leu or Leu-Val-Ser-Glu; and n is 0 or 1).

**Revendications**

1. Utilisation du facteur de stimulation des colonies de granulocytes humains pour préparer une composition pharmaceutique destinée à traiter la leucémie myélogène, ledit facteur de stimulation des colonies de granulocytes humains ayant les propriétés physico-chimiques suivantes :
   i) masse moléculaire : 19000 ± 1000, telle que mesurée par électrophorèse sur gel de dodécylsulfate de sodiumpolyacrylamide;
   ii) point isoélectrique : ayant au moins un des trois points isolélectriques pI = 5,5 ± 0,1, pI = 5, 8 ± 0,1 et pI = 6,1 ± 0,1;
   iii) absorption dans l'ultraviolet : ayant une absorption maximum à 280 nm et une absorption minimum à 250 nm;
   iv) séquence d'amino-acides des 21 résidus, à partir de la terminaison N :

```
H₂N - Thr - Pro - Leu - Gly - Pro - Ala - Ser - Ser -
Leu - Pro - Gln - Ser - Phe - Leu - Leu - Lys - Cys -
Leu - Glu - Gln - Val -
```

2. Utilisation du facteur de stimulation des colonies de granulocytes humains pour préparer une composition pharmaceutique destinée à traiter la leucémie myélogène, ledit facteur de stimulation des colonies de granulocytes humains étant représenté par tout ou partie de la séquence d'amino-acides précisée ci-dessous :

```
(Met)_n Thr   Pro   Leu   Gly   Pro   Ala   Ser   Ser   Leu   Pro
     Gln   Ser   Phe   Leu   Leu   Lys   Cys   Leu   Glu   Gln   Val
     Arg   Lys   Ile   Gln   Gly   Asp   Gly   Ala   Ala   Leu   Gln
     Glu   Lys    X    Cys   Ala   Thr   Tyr   Lys   Leu   Cys   His
     Pro   Glu   Glu   Leu   Val   Leu   Leu   Gly   His   Ser   Leu
     Gly   Ile   Pro   Trp   Ala   Pro   Leu   Ser   Ser   Cys   Pro
     Ser   Gln   Ala   Leu   Gln   Leu   Ala   Gly   Cys   Leu   Ser
     Gln   Leu   His   Ser   Gly   Leu   Phe   Leu   Tyr   Gln   Gly
     Leu   Leu   Gln   Ala   Leu   Glu   Gly   Ile   Ser   Pro   Glu
     Leu   Gly   Pro   Thr   Leu   Asp   Thr   Leu   Gln   Leu   Asp
     Val   Ala   Asp   Phe   Ala   Thr   Thr   Ile   Trp   Gln   Gln
     Met   Glu   Glu   Leu   Gly   Met   Ala   Pro   Ala   Leu   Gln
     Pro   Thr   Gln   Gly   Ala   Met   Pro   Ala   Phe   Ala   Ser
     Ala   Phe   Gln   Arg   Arg   Ala   Gly   Gly   Val   Leu   Val
     Ala   Ser   His   Leu   Gln   Ser   Phe   Leu   Glu   Val   Ser
     Tyr   Arg   Val   Leu   Arg   His   Leu   Ala   Gln   Pro
```

(ou x est Leu ou Leu-Val-Ser-Glu et n est égal à 0 ou à 1).

**Patentansprüche**

1. Verwendung eines menschlichen Granulozyten-Kolonie-stimulierenden Faktors zur Herstellung eines Arzneimittels für die Behandlung von myelogener Leukämie, wobei der menschliche Granulozyten-Kolonie-stimulierende Faktor die folgenden physikochemischen Eigenschaften aufweist:

i) Molekulargewicht:
$19\,000 \pm 1000$, gemessen durch Elektrophorese in einem Natriumdodecylsulfat-Polyacrylamidgel;
ii) Isoelektrischer Punkt:
mindestens einen der drei isoelektrischen Punkte, pI = 5,5 ± 0,1, pI = 5,8 ± 0,1 und pI = 6,1 ± 0,1;
iii) Ultraviolettabsorption:
Ein Absorptionsmaximum bei 280 nm und ein Absorptionsminimum bei 250 nm;
iv) Aminosäuresequenz der 21 Reste vom N-terminalen Ende:

```
H_2N - Thr - Pro - Leu - Gly - Pro - Ala - Ser - Ser -
Leu - Pro - Gln - Ser - Phe - Leu - Leu - Lys - Cys -
Leu - Glu - Gln - Val -
```

2. Verwendung eines menschlichen Granulozyten-Kolonie-stimulierenden Faktors zur Herstellung eines Arzneimittels für die Behandlung von myelogener Leukämie, wobei der menschliche Granulozyten-Kolonie-stimulierende Faktor die gesamte oder einen Teil der nachstehend gezeigten Aminosäuresequenz aufweist:

```
(Met)ₙThr  Pro  Leu  Gly  Pro  Ala  Ser  Ser  Leu  Pro
   Gln  Ser  Phe  Leu  Leu  Lys  Cys  Leu  Glu  Gln  Val
   Arg  Lys  Ile  Gln  Gly  Asp  Gly  Ala  Ala  Leu  Gln
   Glu  Lys   X   Cys  Ala  Thr  Tyr  Lys  Leu  Cys  His
   Pro  Glu  Glu  Leu  Val  Leu  Leu  Gly  His  Ser  Leu
   Gly  Ile  Pro  Trp  Ala  Pro  Leu  Ser  Ser  Cys  Pro
   Ser  Gln  Ala  Leu  Gln  Leu  Ala  Gly  Cys  Leu  Ser
   Gln  Leu  His  Ser  Gly  Leu  Phe  Leu  Tyr  Gln  Gly
   Leu  Leu  Gln  Ala  Leu  Glu  Gly  Ile  Ser  Pro  Glu
   Leu  Gly  Pro  Thr  Leu  Asp  Thr  Leu  Gln  Leu  Asp
   Val  Ala  Asp  Phe  Ala  Thr  Thr  Ile  Trp  Gln  Gln
   Met  Glu  Glu  Leu  Gly  Met  Ala  Pro  Ala  Leu  Gln
   Pro  Thr  Gln  Gly  Ala  Met  Pro  Ala  Phe  Ala  Ser
   Ala  Phe  Gln  Arg  Arg  Ala  Gly  Gly  Val  Leu  Val
   Ala  Ser  His  Leu  Gln  Ser  Phe  Leu  Glu  Val  Ser
   Tyr  Arg  Val  Leu  Arg  His  Leu  Ala  Gln  Pro
```

(wobei X Leu oder Leu-Val-Ser-Glu ist und n den Wert 0 oder 1 hat).